# EUROPÄISCHE PATENTSCHRIFT

(11) **EP 3 383 840 B1**
(45) Veröffentlichungstag und Bekanntmachung des Hinweises auf die Patenterteilung: **02.06.2021**
(21) Anmeldenummer: 16802066.7
(22) Anmeldetag: 29.11.2016
(51) Int. Cl.: C07C 263/10, C07C 265/14

(54) **VERFAHREN ZUR HERSTELLUNG VON ISOCYANATEN**
METHOD FOR MAKING ISOCYANATES
PROCÉDÉ DE FABRICATION D'ISOCYANATES

(30) Priorität: 03.12.2015 EP 15197775
(43) Veröffentlichungstag der Anmeldung: 10.10.2018
(73) Patentinhaber: Covestro Intellectual Property GmbH & Co. KG, 51373 Leverkusen (DE)
(72) Erfinder: ZECHLIN, Joachim, 41472 Neuss (DE); LODDENKEMPER, Tim, 41542 Dormagen (DE); STEFFENS, Friedhelm, 51373 Leverkusen (DE); WASTIAN, Dietmar, 41542 Dormagen (DE)
(74) Vertreter: Levpat
(86) Internationale Anmeldenummer: PCT/EP2016/079072
(87) Internationale Veröffentlichungsnummer: WO 2017/093215

(56) Entgegenhaltungen:
- WO-A1-2007/028715
- WO-A1-2009/027232

## Beschreibung

Die vorliegende Erfindung befasst sich mit der Herstellung von Isocyanaten durch Phosgenierung der korrespondierenden primären Amine in der Gasphase, wobei die Eduktströme Amin und Phosgen getrennt durch einen vorerhitzten Inertgasstrom einem Reaktor zugeführt werden. Isocyanate im Sinne der Erfindung sind dabei im weitesten Sinne alle Isocyanate, deren korrespondierende Amine sich im Wesentlichen zersetzungsfrei in die Gasphase überführen lassen, mit Ausnahme von 1,5-Pentandiisocyanat.

Isocyanate werden in großen Mengen hergestellt und dienen hauptsächlich als Ausgangsstoffe zur Herstellung von Polyurethanen.

Die Produktion von Isocyanaten in der Gasphase geht mit der Bildung von spezifischen, unter den Reaktionsbedingungen festen oder gelösten Nebenprodukten einher, wie zum Beispiel Isocyanurate, Biurete, Allophanate, Carbodiimide oder Harnstoffe.

Diese festen Nebenprodukte bilden sich zwar - abhängig von den Reaktionsbedingungen - in geringen Konzentrationen, können aber zu unangenehmen Ablagerungsproblemen in spezifischen Prozessabschnitten führen.

Bei der Produktion von Isocyanaten in der Gasphase hat es sich bewährt, die Reaktanden Amin und Phosgen über statische Mischeinrichtungen, insbesondere über Düsen, miteinander in Kontakt zu bringen. Dies kann beispielsweise so geschehen, dass das Amin durch eine Düse in einen die Düse umgebenden Phosgenstrom "eingedüst" wird. Auch die umgekehrte Vorgehensweise (Eindüsung von Phosgen in einen die Düse umgebenden Aminstrom) ist denkbar; ebenso der Einsatz von Zweistoffdüsen für Amin und Phosgen. In jedem Fall stellt sich das Problem, dass die gasförmigen Reaktanden Amin und Phosgen direkt an der Austrittsöffnung der Düse in Kontakt miteinander kommen. Aus diesem Grunde beginnt die Entstehung der festen Nebenprodukte auch dort und kann zu Ablagerungen an der Austrittsöffnung der Düse führen.

Diese Ablagerungen an der Austrittsöffnung der Düse führen unter den Bedingungen des Gasphasenprozesses zu einer zusätzlichen Verwirbelung und somit radialen Ungleichverteilung des Amins im Phosgen. Diese zusätzlichen Verwirbelungen erlauben es dem Reaktionsgemisch, die Reaktorwandung bereits im Eintrittsbereich des Reaktors zu berühren - unter Ausbildung von festen Ablagerungen auf der Reaktorwand. Als Konsequenz verringert sich der freie Querschnitt des Reaktors bereits im Eintrittsbereich immer weiter, unter Ausbildung eines erhöhten Differenzdruckes, was schließlich eine verfrühte Abschaltung zur Reinigung erfordert.

Bei Ablagerungen an der Austrittsöffnung der Düse ist zusätzlich zum Aspekt der Verkürzung der Reaktorlaufzeit ein verschlechtertes Nebenproduktspektrum zu beobachten, da die zusätzliche Verwirbelung an der Austrittsöffnung der Düse eine unkontrollierte Rückvermischung von Reaktionsgemisch zur Folge hat. Daraus ergibt sich ein nicht mehr exakt definiertes Verweilzeitprofil im Reaktor, was sich ungünstig auf die Bildung von Nebenprodukten auswirkt.

Es ist daher wünschenswert, dass bei der Herstellung von Isocyanaten in der Gasphase die Reaktanden Phosgen und Amin nicht unmittelbar an der Austrittsöffnung der Düse in Kontakt miteinander treten, sondern erst in einiger Entfernung davon.

Eine solche Prozessvariation würde die Entstehung und somit auch die Ablagerung von festen Nebenprodukten an der Düse verringern. Zusätzlich würde eine solche Prozessvariation das Verweilzeitspektrum homogenisieren, was zu einer Verringerung der Nebenproduktbildung führt.

WO 2009/027232 A1 zeigt eine Methode auf, ein Inertgas in einen Spalt zwischen dem Amin- und dem Phosgenstrom zu dosieren. Beschrieben wird darin, dass diese zusätzliche Dosierung von Inertgas den Hauptteil der Durchmischung von Amin- und Phosgenstrom hinreichend verzögert, um die Bildung von Feststoffen auf der Düsenspitze zu vermeiden.

Nachteil dieser Methode ist jedoch, dass das zugegebene Inertgas unweigerlich eine Kühlung der Düse bewirkt, wenn es nicht - wie der Aminstrom selbst - hinreichend überhitzt wird. Solch eine Überhitzung wird jedoch in WO 2009/027232 A1 nicht offenbart.

Die internationale Patentanmeldung WO 2016/042125 A1 befasst sich ausschließlich mit einem Verfahren zur Herstellung von 1,5-Pentandiisocyanat (PDI).

Im Falle einer Inertgas-induzierten Kühlung der Düse ist ein partielles Kondensieren von Amin an der Innenwandung der Düse wahrscheinlich. Dies gilt insbesondere bei bereits erhöhtem Differenzdruck über den Reaktor, was nach längerer Reaktor-Laufzeit bereits eine erhöhte Kondensationstemperatur für das Amin zur Folge hat. Partiell kondensierendes Amin würde als Tröpfchen aus der Düse ausgetragen werden, die wiederum nach einiger Zeit an der Düsenmündung Ablagerungen ausbilden - durch thermische Belastung allein oder aber durch unvollständige Reaktion mit Phosgen, dass in geringen Konzentrationen auch an der Düsenmündung vorliegt.

Es bestand daher weiterhin ein Bedarf an einer einfachen und kostengünstigen Modifikation des Prozesses zur Herstellung von Isocyanaten in der Gasphase mit ausreichend verringerter Tendenz zur Ablagerung von Feststoffen an der Düse, die die spezifischen Nachteile der Verfahren des Standes der Technik vermeidet.

Überraschend wurde nun gefunden, dass sich die Ablagerungstendenz von festen Nebenprodukten an der Düse drastisch verringern lässt, wenn man das Amin unter speziellen Bedingungen - die insbesondere dadurch gekennzeichnet sind, dass der gasförmige Inertstoffstrom auf eine Temperatur von 100 °C bis 500 °C, bevorzugt 150 °C bis 450 °C, besonders bevorzugt 150 °C bis 400 °C erhitzt wird - wie nachfolgend beschrieben in der Gasphase mit Phosgen umsetzt. Gleichzeitig konnte überraschenderweise gezeigt werden, dass die Ausbeute des Prozesses unter Anwendung der nachfolgend beschriebenen Bedingungen höher ist als unter Anwendung der Bedingungen, die im Stand der Technik beschrieben werden.

Gegenstand der vorliegenden Erfindung ist ein Verfahren zur kontinuierlichen Herstellung eines Isocyanats **(4),** das nicht 1,5-Pentandiisocyanat ist, durch Umsetzung des korrespondierenden primären Amins (**1**) mit Phosgen (**2**) in Gegenwart eines Inerstoffs (**3**), umfassend die folgenden Schritte:
(i) Bereitstellung eines das primäre Amin umfassenden Gasstroms (**10**) einer Temperatur von 200 °C bis 430 °C, bevorzugt 250 °C bis 420 °C, besonders bevorzugt 250 °C bis 400 °C;
(ii) Bereitstellung eines phosgenhaltigen Gasstroms (**20**) einer Temperatur von 200 °C bis 430 °C, bevorzugt 250 °C bis 420 °C, besonders bevorzugt 250 °C bis 400 °C;
(iii) Bereitstellung eines gasförmigen Inertstoffstroms (**30**) einer Temperatur von 100 °C bis 500 °C, bevorzugt 150 °C bis 450 °C, besonders bevorzugt 150 °C bis 400 °C;
(iv) Zuführen der Ströme **10**, **20** und **30** aus den Schritten (i) bis (iii) in einen Reaktor (**2000**) zur Umsetzung des primären Amins (**1**) mit dem Phosgen (**2**) in der Gasphase, wobei
   a) der Reaktor (**2000**) strömungstechnisch mit einem mindestens doppelwandigen Rohr (**1000** - auch als "Ring- oder Trennspaltdüse" bezeichnet), umfassend eine den Innenteil (**1100**) des Rohres umschließende erste Wandung (**1200**) und eine mit der Außenseite der ersten Wandung einen Spalt (**1300**) ausbildende zweite Wandung (**1400**), verbunden ist;
   b) entweder der Strom **10** oder der Strom **20**, bevorzugt Strom **10**, durch den Innenteil (**1100**) des mindestens doppelwandigen Rohres in den Reaktor geführt wird;
   c) der Strom **30** durch den Spalt **1300** zwischen der ersten Wandung und der zweiten Wandung des mindestens doppelwandigen Rohres in den Reaktor geführt wird;
   d) derjenige der Ströme **10** und **20**, der nicht durch den Innenteil des doppelwandigen Rohres geführt wird, bevorzugt also Strom **20**, so an der Außenseite der zweiten Wandung (**1400**) des mindestens doppelwandigen Rohres vorbei geführt wird, dass er am Ende des mindestens doppelwandigen Rohres mit dem Strom **30** in Kontakt tritt;
(v) Aufarbeitung des im Reaktor (**2000**) gebildeten gasförmigen Rohproduktstroms (**40**) unter Erhalt des gewünschten Isocyanats (**4**),
   wobei die Temperatur des in Schritt (ii) bereitgestellten gasförmigen phosgenhaltigen Stroms (**20**) auf einen Wert eingestellt wird, der unterhalb des Siedepunktes des zu phosgenierenden primären aromatischen Amins (**1**) unter den Bedingungen in Schritt (iv) liegt.

Die Erfindung eignet sich zur Herstellung aller Isocyanate, deren korrespondierende Amine sich im Wesentlichen zersetzungsfrei in die Gasphase überführen lassen. Von der Erfindung jedoch nicht erfasst ist die Herstellung von 1,5-Pentandiisocyanat. Insbesondere kann das Isocyanat (**4**) ausgewählt werden aus (**I**) der Gruppe bestehend aus Xylylendiisocyanat, Hexamethylendiisocyanat, Isophorondiisocyanat, Phenylisocyanat, Methylendiphenylmethandiisocyanat und Toluylendiisocyanat. Besonders bevorzugt ist das Isocyanat (**4**) ausgewählt aus (**II**) der Gruppe bestehend aus Xylylendiisocyanat, Hexamethylendiisocyanat und Toluylendiisocyanat. Ganz besonders bevorzugt ist das Isocyanat (**4**) ausgewählt aus (**III**) der Gruppe bestehend aus Toluylendiisocyanat und Hexamethylendiisocyanat. Außerordentlich besonders bevorzugt ist (**IV**) die Herstellung von Toluylendiisocyanat (fortan TDI, **4a**) durch Phosgenierung von Toluylendiamin (fortan TDA, **1a**). Die vorstehend beschriebenen Gruppen (**I**), (**II**), (**III**) und (**IV**) an bevorzugten Isocyanaten (**4**) sind jeweils als *abschließende* Listen zu verstehen, d. h. in der jeweiligen bevorzugten Ausführungsform ist das Isocyanat (**4**) *ausschließlich* aus den Mitgliedern der jeweiligen Gruppe ausgewählt.

Nachfolgend werden Ausführungsformen der Erfindung beschrieben. Verschiedene Ausführungsformen können dabei beliebig miteinander kombiniert werden, sofern sich für den Fachmann aus dem Zusammenhang nicht das Gegenteil ergibt.

Die **Phosgenierung von Aminen in der Gasphase** an sich ist bekannt und kann beispielsweise erfolgen wie beschrieben in EP 0 289 840 B1, EP 1 319 655 A2, EP 1 555 258 A1, EP 1 275 639 A1, EP 1 275 640 A1, EP 1 449 826 A1, EP 1 754 698 B1, DE 10 359 627 A1 oder in der deutschen Patentanmeldung DE 10 2005 042392 A1.

In der bevorzugten Ausführungsform der Phosgenierung von **TDA (1a)** wird vorzugsweise TDA mit einer per Gaschromatographie bestimmten Reinheit von > 99,5 % und einem per Karl-Fischer-Titration bestimmten Wassergehalt von < 300 ppm. Die Herstellung von TDA ist aus dem Stand der Technik bekannt. Es spielt für die Erfindung keine Rolle, ob das TDA aus petrochemisch basierten oder sog. "biobasierten" Rohstoffen stammt. Dies gilt selbstverständlich auch für andere primäre Amine **(1).**

Vor der Durchführung des erfindungsgemäßen Verfahrens wird das **primäre Amin** (**1**) verdampft und auf eine Temperatur von 200 °C bis 430°C, bevorzugt von 250 °C bis 420 °C, besonders bevorzugt von 250 °C bis 400 °C, erhitzt und dem Reaktor (**2000**), der bevorzugt als Rohreaktor ausgestaltet ist, zugeführt. Dabei wird dem primären Amin bevorzugt ein Inertstoff (**3**) wie Stickstoff, He, Ar oder die Dämpfe eines inerten Lösungsmittels, z. B. eines aromatischen Kohlenwasserstoffes mit oder ohne Halogensubstitution, wie z. B. Chlorbenzol, ortho-Dichlorbenzol, Toluol, Chlortoluol, Xylol, Chlornaphtalin oder Decahydrodronaphtalin, bevorzugt Chlorbenzol oder ortho-Dichlorbenzol, insbesondere bevorzugt ortho-Dichlorbenzol, beigemischt. Der gasförmige Strom aus Amin und ggf. vorhandenen gasförmigen Beimischungen wird als Gasstrom **10** bezeichnet. Die oben genannten Temperaturen gelten im Falle des Vorhandenseins von Beimischungen für den gesamten Strom **10**.

Das bei der Phosgenierung verwendete **Phosgen** (**2**) wird vor der Einspeisung in den Reaktor (**2000**) ebenfalls auf eine Temperatur von 200 °C bis 430°C, bevorzugt von 250 °C bis 420 °C, besonders bevorzugt von 250 °C bis 400 °C, erhitzt. Auch dem Phosgen können die o. g. inerten Stoffe beigemischt werden. Der Strom aus gasförmigen Phosgen und ggf. vorhandenen gasförmigen Beimischungen wird als Gasstrom **20** bezeichnet. Die oben genannten Temperaturen gelten im Falle des Vorhandenseins von Beimischungen für den gesamten Strom **20**.

Erfindungsgemäß wesentlich ist, dass Aminstrom **10** und Phosgenstrom **20** dem Reaktor so zugeführt werden, dass diese nicht unmittelbar nach Austritt aus dem jeweiligen Zuführungsrohr sofort miteinander in Kontakt treten. Erfindungswesentlich ist ebenfalls, dass diese Vermeidung des sofortigen Inkontaktretens so erfolgt, dass eine unerwünschte Abkühlung der Reaktanden im getrennten und/oder vermischten Zustand unterbleibt. Hierzu ist es wesentlich, einen Inertgasstrom (**30**) bereitzustellen, der die gleichen inerten Stoffe, wie sie für die Verdünnung des Amin- und/oder des Phosgenstroms geeignet sind, und zuvor beschrieben wurden, umfasst. Dieser Inertgasstrom wird nun erfindungsgemäß auf eine Temperatur von 100 °C bis 500°C, bevorzugt 150 °C bis 450 °C, besonders bevorzugt 150 °C bis 400 °C erhitzt und dem Reaktor wie nachstehend beschrieben zugeführt.

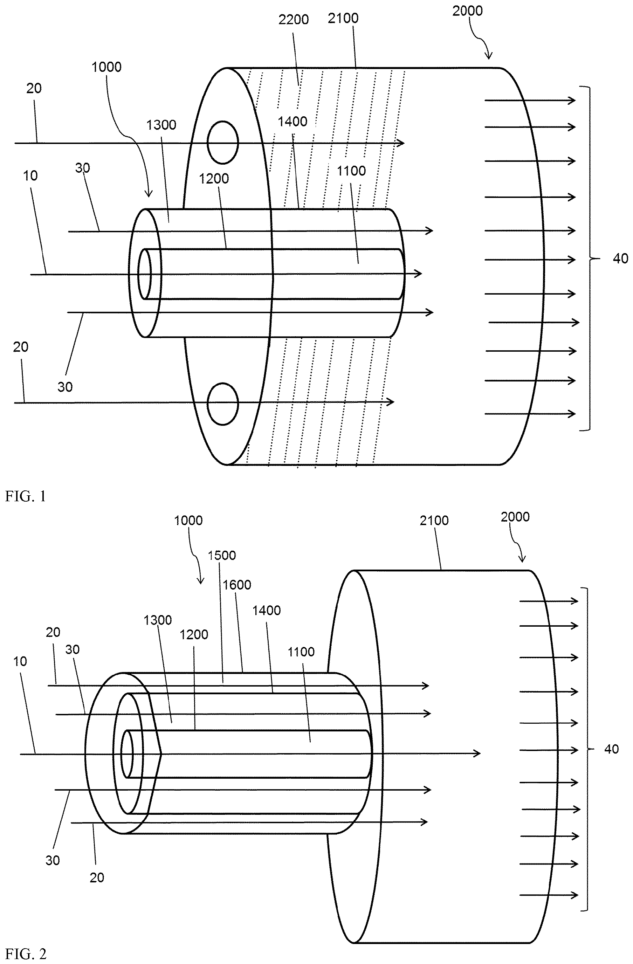

In einer bevorzugten Ausführungsform weist das **Rohr** (**1000**) genau zwei Wandungen (**1200**, **1400**) auf und ragt so in den Reaktor hinein, dass die Innenseite der Wandung (**2100**) des Reaktors (**2000**) und die Außenseite der zweiten Wandung (**1400**) des Rohres einen Spalt (**2200**) bilden, durch den in Schritt (iv) d) der Strom **10** bzw. **20** geführt wird. In dieser Ausführungsform bildet also die Reaktorinnenwand neben der Außenseite der zweiten Wandung (**1400**) die zweite Begrenzung des Stromes gemäß (iv) d). Diese Ausführungsform ist in **FIG. 1** dargestellt. Die erfindungsgemäße separate Vor-Erhitzung des zusätzlich dosierten Inertgasstromes (**30**) führt zur gewünschten Vermeidung von Wärmeverlusten insbesondere an dem Innenteil (**1100**) des mindestens doppelwandigen Rohres (**1000**). In der bevorzugten, in FIG. 1 gezeigten Ausführungsform, in welcher der Amingasstrom (**10**) durch das den Innenteil (**1100**) der Einrichtung **1000** geführt wird, wird der Amingasstrom (**10**) in Form eines Zylindermantels vom Inertgasstrom (**30**) umhüllt. Dies ermöglicht im Gegensatz zum Stand der Technik eine adiabate (d. h. in diesem Zusammenhang ohne signifikante Wärmeverluste) Zuführung des Amingasstroms (**10**) zum Reaktor (**2000**). Die adiabate Amin-Zuführung unterdrückt die Kondensation von Amin an der inneren Wandung des Innenteils **(1100),** wodurch Ablagerungen an der Austrittsöffnung des Innenteils (**1100**) zuverlässig vermieden werden. Die in FIG. 1 gezeigte Zuführung des Amingasstroms (**10**) durch den Innenteil **1100** und die des Phosgengasstroms (**20**) durch den Spalt **2200** ist zwar aus den genannten Gründen bevorzugt, aber nicht zwingend, kann also auch vertauscht werden. Die in FIG. 1 gezeigte Anordnung, in welcher die beiden Wandungen (**1200**, **1400**) des doppelwandigen Rohres (**1000**) gleich lang sind, ist nicht zwingend, aber bevorzugt. Die Wandungen (**1200**, **1400**) können auch unterschiedlich lang sein, wobei es dann bevorzugt ist, dass die innere Wandung (**1200**) länger ist als die äußere (**1400**).

In einer anderen bevorzugten Ausführungsform umfasst das Rohr (**1000**) zusätzlich eine dritte Wandung (**1600**), deren Innenseite mit der Außenseite der zweiten Wandung (**1400**) einen Spalt (**1500**) ausbildet, durch welchen in Schritt (iv) d) der Strom **10** bzw. **20** geführt wird. Diese Ausführungsform ist in **FIG. 2** dargestellt. Die dort gezeigte Zuführung des Amingasstroms (**10**) durch das innerste Rohr und die des Phosgengasstroms (**20**) durch den Spalt **1500** ist zwar bevorzugt, aber nicht zwingend, kann also auch vertauscht werden. Da in dieser Ausführungsform der äußere Ringspalt (**1500**) im Gegensatz zur zuvor beschriebenen Ausführungsform nicht durch die Reaktorinnenwand nach außen begrenzt wird, ist es möglich, das Rohr (1000) direkt an die Eintrittsöffnung des Reaktors (**2000**) anzuflanschen. Es ist aber auch in dieser Ausführungsform möglich, das Rohr (**1000**) in den Reaktor (**2000**) hineinragen zu lassen. Auch in dieser Ausführungsform ist es bevorzugt (aber nicht zwingend), den Amingasstrom (**10**) durch den Innenteil (**1100**) zu führen, wie zuvor für die Ausführungsform gemäß FIG. 1 beschrieben. Die in FIG. 2 gezeigte Anordnung, in welcher die drei Wandungen (**1200**, **1400**, **1600**) des Rohres (**1000**) gleich lang sind, ist nicht zwingend, aber bevorzugt. Die Wandungen (**1200**, **1400**, **1600**) können auch unterschiedlich lang sein, wobei es dann bevorzugt ist, dass die innere Wandung (**1200**) länger ist als die äußeren (**1400**, **1600**).

Unabhängig von der genauen Art, wie der äußere Ringspalt realisiert wird, ist es bevorzugt, dass der **Reaktor** (**2000**) eine zylindrische oder konische Form hat (Rohrreaktor), wobei das mindestens doppelwandige Rohr (**1000**) zentrisch in Richtung der Rotationsachse des Reaktors (**2000**) ausgerichtet ist. Bevorzugt werden im erfindungsgemäßen Verfahren Rohrreaktoren ohne Einbauten und ohne sich bewegende Teile im Innern des Reaktors (**2000**) verwendet. Die Rohrreaktoren bestehen im Allgemeinen aus Stahl, Glas, legiertem oder emailliertem Stahl und sind so dimensioniert, dass unter den Verfahrensbedingungen eine vollständige Umsetzung des primären Amins (**1**) mit dem Phosgen ermöglicht wird. Die Gasströme werden wie oben beschrieben über ein mindestens doppelwandiges Rohr (**1000**) an einem Ende des Rohrreaktors in diesen eingeleitet. In der Mischzone nach dem mindestens doppelwandigen Rohr (**1000**) wird bevorzugt eine Temperatur im Bereich von 300 °C bis 480 °C, vorzugsweise 350 °C bis 450 °C, gehalten, wobei diese Temperatur gegebenenfalls durch Beheizung des Rohreaktors über dessen ganze Länge aufrecht erhalten werden kann.

In allen Ausführungsformen ist es bevorzugt, den ggf. durch ein Inertstoff verdünnten, vorerhitzten gasförmigen Amin-Strom (**10**) mit einer mittleren Strömungsgeschwindigkeit von 10 m/s bis 150 m/s, vorzugsweise 10 m/s bis 100 m/s, dem Reaktor (**2000**) gemäß (iv) b) oder (iv) d), bevorzugt gemäß (iv) b), zuzuführen. Ebenfalls in allen Ausführungsformen ist es bevorzugt, das ggf. durch einen Inertstoff verdünnte, vorerhitzte gasförmige Phosgen (**20**) dem Reaktor (**2000**) mit einer mittleren Strömungsgeschwindigkeit von mindestens 1 m/s, vorzugsweise von 5 m/s bis 15 m/s, zuzuführen.

In allen Ausführungsformen ist der Inertstoff (**3**) bevorzugt ausgewählt aus der Gruppe bestehend aus Stickstoff, Edelgasen, inerten Lösungsmitteln und Mischungen der vorgenannten Stoffe. Geeignete Lösungsmittel sind beispielsweise aromatische Kohlenwasserstoffe mit oder ohne Halogensubstitution, wie z. B. Chlorbenzol, ortho-Dichlorbenzol, Toluol, Chlortoluol, Xylol, Chlornaphtalin oder Decahydrodronaphtalin. Bevorzugt sind Chlorbenzol und ortho-Dichlorbenzol, insbesondere bevorzugt ist ortho-Dichlorbenzol. Werden Amin (**1**) und/oder Phosgen (**2**) mit einem Inertstoff verdünnt, so wird hierzu bevorzugt der gleiche Inertstoff (**3**) verwendet, der vorerhitzt und gasförmig als Strom **30** durch den Spalt **1300** geführt wird. Der gasförmige Inertstoffstrom (**30**) wird bevorzugt dem Reaktor (**2000**) mit einer mittleren Strömungsgeschwindigkeit von 10 m/s bis 150 m/s, vorzugsweise von 10 m/s bis 100 m/s, zugeführt.

Die separate Erhitzung des Inertgasstroms erlaubt die erfindungsgemäße Reaktionsführung, bei der die Temperatur des in Schritt (ii) bereitgestellten gasförmigen phosgenhaltigen Stroms (**20**) auf einen Wert eingestellt wird, der unterhalb des Siedepunktes des zu phosgenierenden primären aromatischen Amins (**1**) unter den Bedingungen in Schritt (iv) liegt, und insbesondere bis zu 80 °C unterhalb des Siedepunkts liegt. Dies gilt insbesondere für die bevorzugte Ausführungsform, in welcher der Amingasstrom (**10**) durch den Innenteil (**1100**) des mindestens doppelwandigen Rohres (**1000**) geführt wird. In einem solchen Fall ist durch die Ummantelung des Innenteils (**1100**) mit dem heißen Inertgas keine
Kondensation von Amin (**1**) an der Innenseite der Wandung **1200** zu befürchten. Die bei der Vermischung von Amingasstrom (**10**) und Phosgengasstrom (**20**) oft entstehenden feinen Kondensations-Tröpfchen werden in dieser Ausführungsform durch die entstehende Reaktionswärme rasch genug verdampft, um eine vollständige Reaktion innerhalb der durch die Reaktorgeometrie gegebenen Verweilzeit zu ermöglichen.

Der Vorteil bei einer solchen alternativen Reaktionsführung liegt darin, dass weniger Wärme in den Reaktor (**2000**) eingebracht werden muss. Die dadurch gesenkte Hot-Spot-Temperatur wirkt sich positiv auf die Ausbeute aus. Zudem muss weniger Energie für die Phosgen-Überhitzung sowie für die Kühlung nach erfolgter Reaktion aufgebracht werden.

Die in den Reaktor (**2000**) geführten Mengenflüsse der Ströme **10** und **20** werden bevorzugt so gewählt, dass der molare Überschuss an Phosgen gegenüber primären Aminogruppen 30 % bis 350 %, bevorzugt 60 % bis 250 % beträgt.

Bevorzugt werden die in den Schritten (i), (ii) und (iii) bereitgestellten Ströme **10**, **20** und **30** dem Reaktor (**2000**) in Schritt (iv) unabhängig voneinander jeweils unter einem (absoluten) Druck von 400 mbar bis 3.000 mbar, bevorzugt von 400 mbar bis 2.000 mbar, besonders bevorzugt von 800 mbar bis 1.800 mbar, zugeführt. Bevorzugt liegt der (absolute) Druck der Reaktionsmischung an der Austrittsöffnung des Reaktors (**2000**) im Bereich von 400 mbar bis 2.000 mbar, bevorzugt 750 mbar bis1.500 mbar, wobei durch Aufrechterhaltung eines geeigneten Differenzdrucks eine gesamte Strömungsgeschwindigkeit innerhalb des Reaktors (**2000**) von 2,0 m/s bis 100 m/s, bevorzugt 5,0 m/s bis 50 m/s eingehalten wird.

Unter diesen Voraussetzungen herrscht innerhalb des Reaktors (**2000**) im Allgemeinen eine Pfropfenströmung vor, die eine gleichmäßige Verweilzeit des Reaktionsgemisches im Reaktor sicherstellt. Die Verweilzeit beträgt 1,0 s bis 10 s, bevorzugt 2,0 s bis 7,0 s. Die Verweilzeit berechnet sich aus dem zeitlichen Durchsatz der Eduktströme, der Reaktor-Dimensionierung und den Reaktionsparametern Druck und Temperatur.

Nach der erfolgten Phosgenierungsreaktion im Reaktor (**2000**) wird das den Reaktor kontinuierlich verlassende, gasförmige Gemisch von Chlorwasserstoffgas und überschüssigem Phosgen befreit und das gebildete Isocyanat (**4**) isoliert. Zu diesem Zweck wird bevorzugt zunächst in Schritt (v) der im Reaktor (**2000**) erhaltene gasförmige Rohproduktstrom (**40**) durch Kontakt mit einer inerten Flüssigkeit (**50**) so abgekühlt, dass ein flüssiger, das gewünschte Isocyanat (**4**) enthaltender Strom **(41)** und ein gasförmiger, Chlorwasserstoff und nicht umgesetztes Phosgen enthaltender Strom (**60**) erhalten werden. Dabei wird die Temperatur der inerten Flüssigkeit (**50**) bevorzugt so gewählt, dass diese einerseits oberhalb der Zersetzungstemperatur des dem Isocyanat (**4**) entsprechenden Carbaminsäurechlorids und andererseits unterhalb der Kondensationstemperatur des Isocyanats (**4**) und vorzugsweise auch des gegebenenfalls in der Dampfform als Verdünnungsmittel mitverwendeten Lösungsmittels liegt, sodass Isocyanat (**4**) und Hilfslösungsmittel kondensieren, während überschüssiges Phosgen, Chlorwasserstoff und gegebenenfalls als Verdünnungsmittel mitverwendetes Inertgas die Kondensationsstufe gasförmig durchlaufen. Bevorzugt wird die inerte Flüssigkeit (**50**) bei einer Temperatur von 100 °C bis 250 °C, vorzugsweise 120 °C bis 190 °C eingesetzt. Als inerte Flüssigkeit (**50**) wird bevorzugt Chlorbenzol, ortho-Dichlorbenzol, Toluol, Chlortoluol, Xylol, Chlornaphtalin oder Decahydrodronaphtalin, besonders bevorzugt Chlorbenzol oder ortho-Dichlorbenzol, ganz besonders bevorzugt ortho-Dichlorbenzol eingesetzt. Besteht der Inertgasstrom (**30**) aus den Dämpfen eines der vorgenannten inerten Lösungsmittel, so wird zweckmäßigerweise bevorzugt als inerte Flüssigkeit (**50**) das gleiche Lösungsmittel in flüssiger Form eingesetzt.

Denkbare Methoden der selektiven Kondensation des gebildeten Isocyanats (**4**) aus dem den Reaktor (2000) verlassenden Gasgemisch (**41**) sind beispielsweise das Durchleiten des Gasgemisches durch die inerte Flüssigkeit (**50**) oder das Eindüsen der inerten Flüssigkeit (**50**) (Lösungsmittelnebel) in den Gasstrom **41** (sog. "Quenche").

Das die Kondensationsstufe zur Gewinnung des Isocyanats (**4**) durchlaufende Gasgemisch (**60**), das bevorzugt unter einem (absoluten) Druck von 400 mbar bis 2.000 mbar steht, wird anschließend in an sich bekannter Weise von überschüssigem Phosgen befreit (Phosgenrückgewinnung). Dies kann mittels einer Kühlfalle, Absorption in einem bei einer Temperatur von -10 °C bis 8 °C gehaltenen inerten Lösungsmittel (bevorzugt das gleiche Lösungsmittel wie die inerte Flüssigkeit **50**) oder durch Adsorption und Hydrolyse an Aktivkohle erfolgen.

Das die Phosgen-Rückgewinnungsstufe durchlaufende Chlorwasserstoffgas kann in an sich bekannter Weise zur Rückgewinnung des zur Phosgensynthese erforderlichen Chlors rezykliert werden.

Die Reindarstellung des Isocyanats (**4**) erfolgt vorzugsweise durch destillative Aufarbeitung des flüssigen, Lösungsmittel enthaltenden Stroms **41.**

Die Vorteile des erfindungsgemäßen Verfahrens sind:
a) Vermeidung von festen Ablagerungen in der Amin-Zuführung (d. h. bevorzugt an der Innenseite der Wandung **1200**) sowie an der Innenwand des Reaktors (**2000**) und somit Erhöhung der Reaktorlaufzeit.
b) Geringe Nebenproduktbildung und dadurch eine geringere Produktion von Isocyanat-Rückstand, was die zu verwertenden Abfallmengen deutlich reduziert.

Nachfolgendes Beispiel illustriert die Erfindung am Beispiel der Phosgenierung von TDA (**1a**) zu TDI (**4a**).

### Beispiele

### Beispiel 1 (erfindungsgemäß - siehe FIG. 1):

In einem Gasphasenreaktor (**2000**) wurden 8,8 kg/h gasförmiges TDA (**10**; 380 °C, 1600 mbar) und 42,8 kg/h gasförmiges Phosgen (**20**; 310 °C, 1600 mbar) mittels einer Ringspaltdüse (**1000**) gemischt und zur Reaktion gebracht. Erfindungsgemäß wurden 0,587 kg/h Stickstoff (**30**) auf 240 °C vorgeheizt und in den Spalt (**1300**) zwischen Amin- und Phosgenzufuhr dosiert. Nach 92 Stunden Laufzeit wurde die Eduktdosierung gestoppt und der Reaktor abgekühlt. Die Begutachtung der ausgebauten Ringspaltdüse (**1000**) ergab, dass diese praktisch frei von festen Rückständen war.

## Patentansprüche

1. Verfahren zur kontinuierlichen Herstellung eines Isocyanats (**4**), das nicht 1,5-Pentandiisocyanat ist, durch Umsetzung des korrespondierenden primären Amins (**1**) mit Phosgen (**2**) in Gegenwart eines Inerstoffs (**3**), umfassend die folgenden Schritte:
(i) Bereitstellung eines das primäre Amin umfassenden Gasstroms (**10**) einer Temperatur von 200 °C bis 430 °C, bevorzugt 250 °C bis 420 °C, besonders bevorzugt 250 °C bis 400 °C;
(ii) Bereitstellung eines phosgenhaltigen Gasstroms (**20**) einer Temperatur von 200 °C bis 430 °C, bevorzugt 250 °C bis 420 °C, besonders bevorzugt 250 °C bis 400 °C;
(iii) Bereitstellung eines gasförmigen Inertstoffstroms (**30**) einer Temperatur von 100 °C bis 500 °C, bevorzugt 150 °C bis 450 °C, besonders bevorzugt 150 °C bis 400 °C;
(iv) Zuführen der Ströme **10**, **20** und **30** aus den Schritten (i) bis (iii) in einen Reaktor (**2000**) zur Umsetzung des primären Amins (**1**) mit dem Phosgen (**2**) in der Gasphase, wobei
a) der Reaktor (**2000**) strömungstechnisch mit einem mindestens doppelwandigen Rohr (**1000**), umfassend eine den Innenteil (**1100**) des Rohres umschließende erste Wandung (**1200**) und eine mit der Außenseite der ersten Wandung einen Spalt (**1300**) ausbildende zweite Wandung (**1400**), verbunden ist;
b) entweder der Strom **10** oder der Strom **20** durch den Innenteil (**1100**) des mindestens doppelwandigen Rohres in den Reaktor geführt wird;
c) der Strom **30** durch den Spalt **1300** zwischen der ersten Wandung und der zweiten Wandung des mindestens doppelwandigen Rohres in den Reaktor geführt wird;
d) derjenige der Ströme **10** und **20**, der nicht durch den Innenteil des doppelwandigen Rohres geführt wird, so an der Außenseite der zweiten Wandung (**1400**) des mindestens doppelwandigen Rohres vorbei geführt wird, dass er am Ende des mindestens doppelwandigen Rohres mit dem Strom **30** in Kontakt tritt;
(v) Aufarbeitung des im Reaktor (**2000**) gebildeten gasförmigen Rohproduktstroms (**40**) unter Erhalt des gewünschten Isocyanats (**4**),
wobei die Temperatur des in Schritt (ii) bereitgestellten gasförmigen phosgenhaltigen Stroms (**20**) auf einen Wert eingestellt wird, der unterhalb des Siedepunktes des zu phosgenierenden primären aromatischen Amins (**1**) unter den Bedingungen in Schritt (iv) liegt.

2. Verfahren gemäß Anspruch 1, bei dem das Isocyanat (**4**) ausgewählt ist aus der Gruppe bestehend aus Xylylendiisocyanat, Hexamethylendiisocyanat, Isophorondiisocyanat, Phenylisocyanat, Methylendiphenylmethandiisocyanat und Toluylendiisocyanat, bevorzugt aus der Gruppe bestehend aus Xylylendiisocyanat, Hexamethylendiisocyanat und Toluylendiisocyanat, besonders bevorzugt aus der Gruppe bestehend aus Toluylendiisocyanat und Hexamethylendiisocyanat.

3. Verfahren gemäß Anspruch 1 oder 2, bei dem das Rohr (**1000**) genau zwei Wandungen (**1200**, **1400**) hat und so in den Reaktor hineinragt, dass die Innenseite der Wandung (**2100**) des Reaktors (**2000**) und die Außenseite der zweiten Wandung (**1400**) des Rohres einen Spalt (**2200**) bilden, durch den in Schritt (iv) d) der Strom **10** bzw. **20** geführt wird.

4. Verfahren gemäß Anspruch 1 oder 2, bei dem das Rohr (**1000**) zusätzlich eine dritte Wandung (**1600**) umfasst, deren Innenseite mit der Außenseite der zweiten Wandung (**1400**) einen Spalt (**1500**) ausbildet, durch welchen in Schritt (iv) d) der Strom **10** bzw. **20** geführt wird.

5. Verfahren gemäß Anspruch 4, bei dem das Rohr (**1000**) an die Eintrittsöffnung des Reaktors (**2000**) angeflanscht ist.

6. Verfahren gemäß Anspruch 4, bei dem das Rohr (**1000**) in den Reaktor (**2000**) hineinragt.

7. Verfahren gemäß einem der Ansprüche 3 bis 6, bei dem der Reaktor (**2000**) eine zylindrische oder konische Form hat und das Rohr (**1000**) zentrisch in Richtung der Rotationsachse des Reaktors (**2000**) ausgerichtet ist.

8. Verfahren gemäß einem der vorstehenden Ansprüche, bei dem der Strom **10** durch den Innenteil (**1100**) des mindestens doppelwandigen Rohres (**1000**) in den Reaktor (**2000**) geführt wird.

9. Verfahren gemäß einem der vorstehenden Ansprüche, bei dem der Inertstoff (**3**) ausgewählt ist aus der Gruppe bestehend aus Stickstoff, Edelgasen, inerten Lösungsmitteln und Mischungen der vorgenannten Stoffe.

10. Verfahren gemäß einem der vorstehenden Ansprüche, bei dem das Isocyanat Toluylendiiisocyanat ist und bevorzugt das als primäres Amin eingesetzte Toluylendiamin eine per Gaschromatographie bestimmte Reinheit von > 99,5 % und einen per Karl-Fischer-Titration bestimmten Wassergehalt von < 300 ppm hat.

11. Verfahren gemäß einem der vorstehenden Ansprüche, bei dem die in den Reaktor geführten Mengenflüsse der Ströme **10** und **20** so gewählt werden, dass der molare Überschuss an Phosgen gegenüber primären Aminogruppen 30 % bis 350 % beträgt.

12. Verfahren gemäß einem der vorstehenden Ansprüche, bei dem
die in den Schritten (i), (ii) und (iii) bereitgestellten Ströme **10**, **20** und **30** dem Reaktor in Schritt (iv) unabhängig voneinander jeweils unter einem Druck von 400 mbar bis 3.000 mbar, bevorzugt von 400 mbar bis 2.000 mbar, besonders bevorzugt von 800 mbar bis 1.800 mbar, zugeführt werden.

13. Verfahren gemäß einem der vorstehenden Ansprüche, bei dem in Schritt (v) der im Reaktor (**2000**) erhaltene gasförmige Rohproduktstrom (**40**) durch Kontakt mit einer inerten Flüssigkeit (**50**) so abgekühlt wird, dass ein flüssiger, das gewünschte Isocyanat (**4**) enthaltender Strom (**41**) und ein gasförmiger, Chlorwasserstoff und nicht umgesetztes Phosgen enthaltender Strom (**60**) erhalten werden.

14. Verfahren gemäß Anspruch 13, bei dem der gasförmige, Chlorwasserstoff und nicht umgesetztes Phosgen enthaltende Strom (**60**) unter einem Druck von 400 mbar bis 2.000 mbar steht.

15. Verfahren gemäß einem der vorstehenden Ansprüche, bei dem die Temperatur des in Schritt (ii) bereitgestellten gasförmigen phosgenhaltigen Stroms (**20**) auf einen Wert eingestellt wird, der bis zu 80 °C unterhalb des Siedepunkts des zu phosgenierenden primären aromatischen Amins (**1**) unter den Bedingungen in Schritt (iv) liegt.

## Claims

1. Process for continuously preparing an isocyanate (**4**) other than pentane 1,5-diisocyanate, by reacting the corresponding primary amine (**1**) with phosgene (**2**) in the presence of an inert substance (**3**), comprising the following steps:
(i) providing a gas stream (**10**) comprising the primary amine and having a temperature of 200°C to 430°C, preferably 250°C to 420°C, more preferably 250°C to 400°C;
(ii) providing a phosgene-containing gas stream (**20**) having a temperature of 200°C to 430°C, preferably 250°C to 420°C, more preferably 250°C to 400°C;
(iii) providing a gaseous inert substance stream (**30**) having a temperature of 100°C to 500°C, preferably 150°C to 450°C, more preferably 150°C to 400°C;
(iv) feeding the streams **10, 20** and **30** from steps (i) to (iii) into a reactor (**2000**) for reaction of the primary amine (**1**) with the phosgene (**2**) in the gas phase, wherein
a) the reactor (**2000**) has flow connection to an at least twin-wall tube (**1000**) comprising a first wall (**1200**) that encloses the inner portion (**1100**) of the tube and a second wall (**1400**) that forms a gap (**1300**) with the outside of the first wall;
b) either the stream **10** or the stream **20** is guided through the inner portion (**1100**) of the at least twin-wall tube into the reactor;
c) the stream **30** is guided through the gap **1300** between the first wall and the second wall of the at least twin-wall tube into the reactor;
d) that stream **10** or **20** which is not guided through the inner portion of the twin-wall tube is guided past the outside of the second wall (**1400**) of the at least twin-wall tube such that it comes into contact with the stream **30** at the end of the at least twin-wall tube;
(v) working up the gaseous crude product stream (**40**) formed in the reactor (**2000**) to obtain the desired isocyanate **(4),**
wherein the temperature of the gaseous phosgene-containing stream (**20**) provided in step (ii) is adjusted to a value below the boiling point of the primary aromatic amine (**1**) to be phosgenated under the conditions in step (iv) .

2. Process according to Claim 1, in which the isocyanate (**4**) is selected from the group consisting of xylylene diisocyanate, hexamethylene diisocyanate, isophorone diisocyanate, phenyl isocyanate, methylene diphenylmethane diisocyanate and tolylene diisocyanate, preferably from the group consisting of xylylene diisocyanate, hexamethylene diisocyanate and tolylene diisocyanate, more preferably from the group consisting of tolylene diisocyanate and hexamethylene diisocyanate.

3. Process according to Claim 1 or 2, in which the tube **(1000)** has exactly two walls **(1200, 1400)** and projects into the reactor such that the inside of the wall (**2100**) of the reactor (**2000**) and the outside of the second wall (**1400**) of the tube form a gap (**2200**) through which the stream **10** or **20** is guided in step (iv) d).

4. Process according to Claim 1 or 2, in which the tube **(1000)** additionally comprises a third wall **(1600),** the inside of which forms, with the outside of the second wall **(1400),** a gap (**1500**) through which the stream **10** or **20** is guided in step (iv) d).

5. Process according to Claim 4, in which the tube **(1000)** is flanged on to the entry orifice of the reactor (**2000**).

6. Process according to Claim 4, in which the tube **(1000)** projects into the reactor **(2000).**

7. Process according to any of Claims 3 to 6, in which the reactor (**2000**) has a cylindrical or conical shape and the tube (**1000**) is aligned centrally in the direction of the axis of rotation of the reactor **(2000).**

8. Process according to any of the preceding claims, in which the stream **10** is guided through the inner portion (**1100**) of the at least twin-wall tube (**1000**) into the reactor (**2000**).

9. Process according to any of the preceding claims, in which the inert substance (**3**) is selected from the group consisting of nitrogen, noble gases, inert solvents and mixtures of the aforementioned substances.

10. Process according to any of the preceding claims, in which the isocyanate is tolylene diisocyanate and the tolylenediamine used as primary amine preferably has a purity determined by gas chromatography of > 99.5% and a water content determined by Karl Fischer titration of < 300 ppm.

11. Process according to any of the preceding claims, in which the flow rates of the streams **10** and **20** guided into the reactor are chosen such that the molar excess of phosgene over primary amino groups is 30% to 350%.

12. Process according to any of the preceding claims, in which
the streams **10, 20** and **30** provided in steps (i), (ii) and (iii) are each independently fed to the reactor in step (iv) under a pressure of 400 mbar to 3000 mbar, preferably of 400 mbar to 2000 mbar, more preferably of 800 mbar to 1800 mbar.

13. Process according to any of the preceding claims, in which the gaseous crude product stream (**40**) obtained in the reactor (**2000**) in step (v) is cooled down by contact with an inert liquid (**50**) such that a liquid stream (**41**) comprising the desired isocyanate (**4**) and a gaseous stream (**60**) comprising hydrogen chloride and unconverted phosgene are obtained.

14. Process according to Claim 13, in which the gaseous stream (**60**) comprising hydrogen chloride and unconverted phosgene is under a pressure of 400 mbar to 2000 mbar.

15. Process according to any of the preceding claims, in which the temperature of the gaseous phosgene-containing stream (**20**) provided in step (ii) is adjusted to a value up to 80°C below the boiling point of the primary aromatic amine (**1**) to be phosgenated under the conditions in step (iv).

## Revendications

1. Procédé de production en continu d'un isocyanate (4) qui n'est pas du diisocyanate de 1,5-pentane, par réaction de l'aminé primaire correspondante (1) avec du phosgène (2) en présence d'une substance inerte (3), le procédé comprenant les étapes suivantes :
(i) fournir un flux gazeux (10), comprenant l'amine primaire, à une température de 200 °C à 430 °C, de préférence de 250 °C à 420 °C, de manière particulièrement préférée de 250 °C à 400 °C ;
(ii) fournir un flux gazeux, contenant du phosgène (20), à une température de 200 °C à 430 °C, de préférence de 250 °C à 420 °C, de manière particulièrement préférée de 250 °C à 400 °C ;
(iii) fournir un flux de substance inerte gazeuse (30) à une température de 100 °C à 500 °C, de préférence 150 °C à 450 °C, de manière particulièrement préférée 150 °C à 400 °C ;
(iv) amener les flux 10, 20 et 30 des étapes (i) à (iii) jusque dans un réacteur (2000) pour faire réagir l'amine primaire (1) avec le phosgène (2) en phase gazeuse,
a) le réacteur (2000) étant relié fluidiquement à un tube au moins à double paroi (1000), comprenant une première paroi (1200) entourant la partie intérieure (1100) du tube et une deuxième paroi (1400) formant un espace (1300) avec le côté extérieur de la première paroi) ;
b) soit le flux 10, soit le flux 20, qui sont guidés à travers la partie intérieure (1100) du tube au moins à double paroi jusque dans le réacteur ;
c) le flux 30 étant guidé à travers l'espace 1300 ménagé, entre la première paroi et la deuxième paroi du tube au moins à double paroi, jusque dans le réacteur ;
d) celui des flux 10 et 20, qui n'est pas guidé à travers la partie intérieure du tube à double paroi, étant guidé devant le côté extérieur de la deuxième paroi (1400) de l'au moins un tube à double paroi de manière à venir en contact avec le flux 30 à l'extrémité du tube au moins à double paroi ;
(v) traiter le flux de produit brut gazeux (40), formé dans le réacteur (2000), pour obtenir l'isocyanate souhaité (4),
la température du flux gazeux, contenant du phosgène (20) fourni à l'étape (ii), étant réglée à une valeur qui est inférieure au point d'ébullition de l'amine aromatique primaire (1) à phosgéner dans les conditions de l'étape (iv).

2. Procédé selon la revendication 1, dans lequel l'isocyanate (4) est choisi dans le groupe comprenant le diisocyanate de xylylène, le diisocyanate d'hexaméthylène, le diisocyanate d'isophorone, l'isocyanate de phényle, le diisocyanate de méthylènediphénylméthane et le diisocyanate de tolylène, de préférence dans le groupe comprenant le diisocyanate de xylylène, le diisocyanate d'hexaméthylène et le diisocyanate de tolylène, de manière particulièrement préférée dans le groupe comprenant le diisocyanate de tolylène et le diisocyanate d'hexaméthylène.

3. Procédé selon la revendication 1 ou 2, dans lequel le tube (1000) comporte exactement deux parois (1200, 1400) et fait saillie dans le réacteur de sorte que le côté intérieur de la paroi (2100) du réacteur (2000) et le côté extérieur de la deuxième paroi (1400) du tube forment un espace (2200) à travers lequel le flux 10 ou 20 est guidé à l'étape (iv)d).

4. Procédé selon la revendication 1 ou 2, dans lequel le tube (1000) comprend en outre une troisième paroi (1600) dont le côté intérieur forme avec le côté extérieur de la deuxième paroi (1400) un espace (1500) à travers lequel le flux 10 ou 20 est guidé à l'étape (iv)d).

5. Procédé selon la revendication 4, dans lequel le tube (1000) est bridé au niveau de l'ouverture d'entrée du réacteur (2000).

6. Procédé selon la revendication 4, dans lequel le tube (1000) fait saillie dans le réacteur (2000).

7. Procédé selon l'une des revendications 3 à 6, dans lequel le réacteur (2000) a une forme cylindrique ou conique et le tube (1000) est orienté centralement en direction de l'axe de rotation du réacteur (2000).

8. Procédé selon l'une des revendications précédentes, dans lequel le flux 10 est guidé à travers la partie intérieure (1100) du tube au moins à double paroi (1000) jusque dans le réacteur (2000).

9. Procédé selon l'une des revendications précédentes, dans lequel la substance inerte (3) est choisie dans le groupe comprenant l'azote, les gaz rares, les solvants inertes et des mélanges des substances susmentionnées.

10. Procédé selon l'une des revendications précédentes, dans lequel l'isocyanate est le diisocyanate de toluène et de préférence le diamine de toluène utilisé comme amine primaire a une pureté > 99,5 %, déterminée par chromatographie en phase gazeuse, et une teneur en eau < 300 ppm, déterminée par titrage Karl Fischer.

11. Procédé selon l'une des revendications précédentes, dans lequel les flux massiques 10 et 20 introduits dans le réacteur sont choisis de telle sorte que l'excès molaire de phosgène par rapport aux groupements amine primaires soit de 30 % à 350 %.

12. Procédé selon l'une des revendications précédentes, dans lequel les flux 10, 20 et 30, fournis aux étapes (i), (ii) et (iii), sont amenés au réacteur à l'étape (iv) indépendamment les uns des autres à chaque fois sous une pression de 400 mbar à 3000 mbar, de préférence de 400 mbar à 2000 mbar, de manière particulièrement préférée de 800 mbar à 1800 mbar.

13. Procédé selon l'une des revendications précédentes, dans lequel à l'étape (v) le flux de produit brut gazeux (40), obtenu dans le réacteur (2000), est refroidi par contact avec un liquide inerte (50) de façon à obtenir un flux liquide (41), contenant l'isocyanate souhaité (4), et un flux gazeux (60) contenant du chlorure d'hydrogène et du phosgène n'ayant pas réagi.

14. Procédé selon la revendication 13, dans lequel le flux gazeux (60), contenant du chlorure d'hydrogène et du phosgène n'ayant pas réagi, est à une pression de 400 mbar à 2000 mbar.

15. Procédé selon l'une des revendications précédentes, dans lequel la température du flux gazeux (20), contenant du phosgène fourni à l'étape (ii), est réglée à une valeur qui est jusqu'à 80 °C au-dessous du point d'ébullition de l'amine aromatique primaire (1) à phosgéner dans les conditions de l'étape (iv).
